# EUROPEAN PATENT SPECIFICATION

(11) **EP 2 026 768 B1**
(45) Date of publication and mention of the grant of the patent: **28.02.2018**
(21) Application number: 07736412.3
(22) Date of filing: 03.06.2007
(51) Int. Cl.: A61K 9/20, A61K 9/50, A61K 31/4439

(54) **MULTIPLE UNIT PHARMACEUTICAL FORMULATION**
PHARMAZEUTISCHE FORMULIERUNG AUF MEHREREN EINHEITEN
FORMULATION PHARMACEUTIQUE COMPRENANT DES UNITÉS MULTIPLES

(30) Priority: 01.06.2006 US 809942 P
(43) Date of publication of application: 25.02.2009
(73) Proprietor: Dexcel Pharma Technologies Ltd., 38100 Hadera (IL)
(72) Inventor: PENHASI, Adel, 58671 Holon (IL); MOSES-HELLER, Sheera, 30300 Atlit (IL); GOMBERG, Mila, 97482 Jerusalem (IL); AVRAMOFF, Avi, 34403 Haifa (IL)
(74) Representative: Becker Kurig Straus
(86) International application number: PCT/IL2007/000672
(87) International publication number: WO 2007/138606

(56) References cited:
- WO-A-93/25204
- WO-A-96/01624
- DE-A1- 19 752 843
- SHIMIZU T ET AL: "FORMULATION STUDY FOR LANSOPRAZOLE FAST-DISINTEGRATING TABLET. I. EFFECT OF COMPRESSION ON DISSOLUTION BEHAVIOR" CHEMICAL AND PHARMACEUTICAL BULLETIN, PHARMACEUTICAL SOCIETY OF JAPAN, TOKYO, JP, vol. 51, no. 8, August 2003 (2003-08), pages 942-947, XP001170527 ISSN: 0009-2363
- SHIMIZU T ET AL: "FORMULATION STUDY FOR LANSOPRAZOLE FAST-DISINTEGRATING TABLET. II. EFFECT OF TRIETHYL CITRATE ON THE QUALITY OF THE PRODUCTS" CHEMICAL AND PHARMACEUTICAL BULLETIN, PHARMACEUTICAL SOCIETY OF JAPAN, TOKYO, JP, vol. 51, no. 9, October 2003 (2003-10), pages 1029-1035, XP001174705 ISSN: 0009-2363
- SHIMIZU T ET AL: "Formulation Study for Lansoprazole Fast-disintegrating Tablet. III. Design of Rapidly Disintegrating Tablets" CHEMICAL AND PHARMACEUTICAL BULLETIN, PHARMACEUTICAL SOCIETY OF JAPAN, TOKYO, JP, vol. 51, no. 10, 2003, pages 1121-1127, XP003015028 ISSN: 0009-2363

## Description

### FIELD OF THE INVENTION

The present invention relates to a novel formulation for a benzimidazole, and methods of preparation thereof, and in particular, to an individually enteric-coated multiple unit formulation.

### BACKGROUND OF THE INVENTION

Omeprazole, Pantoprazole, Lansoprazole and other derivatives of benzimidazole, which are active proton pump inhibitors and used conventionally for decreasing gastric secretion, are known to be susceptible to degradation and transformation in acid media.

Lansoprazole, 2-[[(3-methyl-4-(2,2,2-trifluoroethoxy)-2-pyridyl) methyl]sulfinyl] benzimidazole is described for example in US Patent Nos. 4,628,098, and 4,689,333 and European Patent No. 174726.

Another popular benzimidazole derivative, Omeprazole, 5-methoxy-2(((4-methoxy-3,5-dimethyl-2-pyridinyl)methyl)sulfinyl)-1H-benzimidazole, is disclosed and described in European Patent No. 5129 and European Patent No. 124495, as well as in numerous other patents and published patent applications.

The susceptibility of these active proton pump inhibitor substances to degradation and transformation in acid media increases the difficulty of preparing a pharmaceutical form designed for oral administration. If the active substance comes into contact with the stomach content, which is a highly acidic medium, these chemical substances become degraded. Thus, these benzimidazoles should be protected both during storage and during their passage through the acidic environment of the stomach.

European Patent No. 237200 discloses one solution, which is to directly coat the solid core containing the benzimidazole with an enteric coating layer.

Enteric coating layers are formed by use of enteric polymers, such as cellulose, vinyl, and acrylic derivatives. These polymers exhibit resistance to gastric fluids, yet are readily soluble or permeable in intestinal fluid. Enteric polymeric materials are primarily weak acids containing acidic functional groups, which are capable of ionization at elevated pH. In the low pH of the stomach, the enteric polymers are unionized, and therefore, insoluble. As the pH increases in the intestinal tract, these functional groups ionize, and the polymer becomes soluble in the intestinal fluids. Thus, an enteric polymeric film coating allows the coated solid to pass intact through the stomach to the small intestine, where the drug is then released for absorption through the intestinal mucosa into the human body where it can exert its pharmacologic effects.

However, this apparent solution to the instability of benzimidazoles caused further complications, in that the alkaline core containing the benzimidazole was found to react with the enteric coating, thereby causing the enteric coating to degrade. A solution to these further complications is disclosed in United Kingdom Patent Application No. 2,189,698, in which the benzimidazole is contained within a solid active core, which is coated first with a subcoating layer and then with an enteric coating layer. The enteric coating layer protects the benzimidazole during the passage through the stomach, while the sub-coating layer protects the enteric coating layer from reacting negatively with the alkaline core containing the benzimidazole.

Oral dosage forms can be classified into two types: single unit and multiple unit. The above documents teach single unit forms comprising a single core, which may comprise a neutral core coated with a layer containing the active ingredient, or an active core in which the active ingredient is admixed with the core excipients.

Multiple-unit dosage forms have been accepted to provide advantages over single unit dosage forms. The multiple-unit dosage forms consist of many small particles, which are contained in a capsule or a tablet. The small particles are mixed with the contents in gastrointestinal tract and are distributed over a large area. Thus, high-local concentration of the drug is avoided, and the risk of local irritations is reduced.

Multiple unit dosage forms are essential where drug excipients or drug-drug physicochemical interaction is possible in a single-unit formulation; they are also known to have less variance in transit time through the gastrointestinal tract than single-unit dosage forms. Multiple-unit forms offer more predictable gastric emptying, which is less dependent on the state of nutrition, a high degree of dispersion in the digestive tract, less absorption variability, and a lesser risk of dose dumping. However, problems arise when enteric coating layered pellets containing acidic susceptible benzimidazoles as an active substance are compressed into tablets.

If the enteric coating layer does not withstand the compression of the pellets into a tablet the susceptible active substance will be destroyed by penetrating acidic gastric juice, i.e. the acid resistance of the enteric coating layer of the pellets will not be sufficient to protect the active ingredient in the tablet after compression. Such problems are typically caused by brittleness of the enteric coating, which causes cracks to form in the coating under the pressure of compression.

Plasticizers are materials having lower molecular weights than those of enteric polymers, and are commonly included in the enteric coating layer to increase separation between the polymer chains, thereby reducing the stiffness and brittleness of the coating layer, thus preventing cracking.

However, the use of a plasticizer in the coating layer is associated with a number of disadvantages. Hydrophobic plasticizers will create problems in enteric coating solution preparation due to poor solubility in aqueous solvents, and can affect the dissolution profile of the finished product. Higher concentrations of plasticizer in the coating generally tends to increase the water vapor permeability, and also to reduce the tensile strength of the coating layer. Higher concentration of plasticizer can also lead to bleeding of the plasticizer, giving an oily feel to the tablet surface. Volatile plasticizers such as propylene glycol may be largely lost due to drying during the coating process.

U.S. Patent No. 5,464,632 teaches a rapidly disintegratable multiparticulate tablet, comprising a plurality of microcrystals or microgranules. The disintegration rate is obtained due to a mixture of excipients or vehicles which comprises at least a disintegrating agent and a swelling agent, which are mixed with the active substance. The crystals or granules may be enteric coated and formed into tablets by compression. This tablet would not be expected to be sufficiently resistant to gastric acid penetration to be suitable for use with a highly acid-sensitive benzimidazole, nor is such an acid-sensitive material taught as a suitable active ingredient.

U.S. Patent No. 6,740,339 teaches rapidly disintegrating solid preparations. U.S. Patent No. 4,786,505 teaches enteric-coated tablets comprising omeprazole, which do not include the use of a plasticizer in the enteric coating. Neither of these documents teaches individually enteric-coated multiple units which are compressed into a tablet.

U.S. Patent Application No. 2004/0213847 teaches an oral pharmaceutical composition in an enteric-coated solid dosage form. Multiple unit dosage forms are not disclosed. Furthermore, the enteric coating in this formulation includes a plasticizer.

U.S. Patent No. 5,985,322 discloses an enteric formulation of the anti-depressant drug, fluoxetine. Again, multiple unit dosage forms are not taught, and the enteric coating is stated as requiring the addition of a plasticizer.

PCT Application No. WO 06/012634 teaches dosage forms with an enterically coated core tablet. Multiple unit dosage forms are not taught, and the enteric coating disclosed in the examples includes a plasticizer.

U.S. Patent Application No. 2002/0142034, PCT Application No. 99/59544 and European Patent Application No. EP 1121103 teach orally disintegratable tablets comprising fine granules, which are coated with an enteric coating layer, and may be compressed into tablets. The tablets include a plasticizer in the enteric coating layer.

U.S. Patent Application No. 2004/1031675 teaches a method of manufacturing a tablet containing coated granules by compression, such that the coating film can be prevented from rupture. Examples of suitable enteric coating layers are given as including at least one plasticizer.

U.S. Patent No. 5,798,120 teaches tablets containing enteric-coated granules, which require the use of a plasticizer.

U.S. Patent Application No. 2006/0018964 teaches a multiparticulate tablet enteric coated particles, and a mixture of tableting excipients, comprising xylitol and/or maltitol, a disintegrating agent, a lubricant and at least one other diluent. The enteric coating includes at least one plasticizer.

U.S. Patent No. 5,753,265 teaches a multiple unit tableted dosage form, comprising individually enteric-coated layered units, compressed into a tablet. The enteric layers are stated as containing pharmaceutically acceptable plasticizers to obtain the desired mechanical properties.

U.S. Patent Application No. 2004/0131675 teaches a method of manufacturing a tablet, which comprises compressing coated granules containing a physiologically active substance, at a temperature exceeding room temperature, whereby the tablet can be prevented from rupture of a part of a coating film of the granules at the time of tablet compression.

U.S. Patent No. 7,041,316 teaches an enteric-coated pharmacological dosage form which comprises a core tablet formed by dry mixing, without using an aqueous solution. The use of a plasticizer in the enteric coating is taught. No mention is made of the use of individually enteric-coated multiple units which are compressed into a tablet. U.S. Patent No. 5,232,706 teaches an oral pharmaceutical preparation for omeprazole, comprising a nucleus, a first coating, and a second, enteric coating. Multiple unit dosage forms are not taught.

U.S. Patent No. 6,733,778 teaches an ompeprazole formulation comprising an active core which is directly enteric coated without the use of a separating layer between the core and the enteric coating. The formulation may further comprise a seal coat containing color, applied over the enteric coating. The use of multiple unit dosage forms is not taught, and the seal coat does not serve to protect the integrity of the enteric coating.

U.S. Patent No. 5,817,338 teaches a multiple unit tableted dosage form of omeprazole, in which pellets or granules are covered with an enteric layer comprising a plasticizer.

U.S. Patent No. 6,780,435 teaches an omeprazole formulation comprising a pellet and a single layer of enteric coating. A seal coating may be applied to the pellets, which does not serve to protect the integrity of the enteric coating. Compression of the pellets to form a tablet is not taught.

U.S. Patent No. 6,576,258 teaches a pharmaceutical formulation for acid-sensitive active substances which are stabilized by anhydrous granulation. The formulation comprises pellets or granules which are compressed into tablets and subsequently coated with an enteric material. The use of particles or granules which are individually coated prior to compression is not taught.

U.S. Patent No. 6,228,400 teaches pharmaceutical formulations for benzimidazole derivatives, comprising granules which may be compressed into tablets. The granules are individually coated with an enteric layer which includes a plasticizer. The use of an outer coating to protect the integrity of the enteric layer is not taught.

U.S. Patent No. 6,551,621 teaches omeprazole microgranules each comprising an active layer and an outer enteric layer. The enteric layer includes a hydrophobic plasticizer. Compression of the microgranules to form a tablet is not taught; nor is the use of an outer layer to preserve the integrity of the enteric coating.

U.S. Patent Nos. 6,136,344, 6,183,776 and 6,132,770 teach pharmaceutical dosage forms comprising an acid susceptible proton pump inhibitor in a multiple unit dosage form, which is enteric-coated. The enteric coating layer has mechanical properties such that the acid resistance of the enteric coated pellets is not significantly affected by compression of the pellets during tableting. The enteric coating comprises a plasticizer.

WO 93/25204 discloses a stable formulation of omeprazole microgranules comprising a neutral core consisting of sugar and starch, characterized in that it comprises an active layer consisting of a dilution of omeprazole in mannitol in substantially equal quantities. The omeprazole microgranules are filled into a capsule.

WO 96/01624 discloses pharmaceutical preparations in the form of a multiple unit tableted dosage form comprising an active substance in the form of an acid labile H⁺K⁺-ATPase inhibitor intended for oral use. The enteric coating contains plasticizers.

DE 197 52 843 discloses pantoprazole and omeprazole tablets and pellets for oral administration comprising: (a) a core containing the active ingredient as an alkali salt and/or with the addition of other alkaline substances; and (b) a core coating of neutralized gastric acid-resistant film former; and optionally (c) one or more additional coatings of gastric acid-resistant film formers.

Drug Dev. Ind. Pharm. 24(8): 737-746 (1998) discusses the compactability of beads for oral dosage forms. Multi-layered beads, consisting of several layers of acetaminophen and polymer coating were studied, having an outer layer of mannitol as a cushioning excipient. Caplets having an outer layer of Avicel PH-101 or polyethylene oxide (PEO), and a center layer of polymer-coated beads are described as exhibiting fracturing of the polymer coating.

Drug Dev. Ind. Pharm. 25(5): 635-652 (1999) studies the prevention or mitigation of polymer coat fracture on compaction of sustained-release beads, without the addition of cushioning excipients. Swellable polymers, such as PEO, were found to prevent polymer coat rupture, but cracks did occur, which were sealed by the PEO. Polymer coatings overcoated with polyethylene glycol and microcrystalline cellulose, with an additional coating of a disintegrant, were found to partially disrupt on compaction. Ethylcellulose-coated beads granulated with cushioning excipients were also found to result in a ruptured polymer coat on compaction.

### SUMMARY OF THE INVENTION

The background art does not teach or suggest a pharmaceutical preparation for benzimidazoles, having individually enterically-coated multiple units, wherein the enteric coating is covered by an outer coating which provides resistance to cracking of the enteric coating during compression, and which is devoid of a plasticizer.

There is thus a widely recognized need for, and it would be highly advantageous to have, multiple unit enteric-coated preparations, particularly for benzimidazoles but optionally for an active ingredient with a bitter or unpleasant taste, which are devoid of at least some of the limitations that are known in the art.

The present invention overcomes these limitations by providing a novel, rapidly orally or extra-orally disintegratable composition for a benzimidazole comprising a multiplicity of enteric-coated units, wherein each of the individual enteric-coated units is entirely coated with an outer layer which protects the integrity of the enteric coating during compression.

According to one aspect of the present invention there is provided a composition comprising a rapidly orally or extra-orally disintegratable tablet having a multiplicity of compressed units, wherein each unit comprises a substrate comprising benzimidazole; an enteric coating, which is devoid of a plasticizer, layered on the substrate; and at least one type of an outer coating layered on said enteric coating and comprising a stress absorber.

According to another embodiment of the present invention, the outer coating protects the integrity of the enteric coating during compression.

According to another embodiment of the present invention, the outer coating prevents direct contact between the units and thus protects the integrity of the enteric coating.

According to another embodiment of the present invention, the outer coating provides protection against humidity and thereby increases chemical stability of the benzimidazole. According to another embodiment of the present invention, the outer coating provides good flowability. According to an additional aspect of the present invention there is provided a method for producing a rapidly orally or extra-orally disintegratable composition comprising: (a) providing a multiplicity of units, wherein each unit comprises a substrate comprising benzimidazole, an enteric coating layered on the substrate, and at least one type of an outer coating layered on said enteric coating, wherein the enteric coating is devoid of any plasticizer and said outer coating comprises a stress absorber; (b) forming a mixture of the multiplicity of units; and (c) compressing the mixture to form a tablet.

According to further features of this embodiment of the method of the present invention, the substrate is optionally produced by dissolving the benzimidazole in an aqueous dispersion and spraying the dispersion onto an inert core. The substrate may be produced by a process of compression, granulation, extrusion or spheronization.

The stress absorber may be, for example, at least one of a polysaccharide or cross-linked polysaccharide, starch, microcrystalline cellulose, ethyl cellulose, a peptide or cross-linked peptide, a protein or cross-linked protein, gelatin or cross-linked gelatin, hydrolyzed gelatin or cross-linked hydrolyzed gelatin, collagen or cross-linked collagen, modified cellulose, polyacrylic acid or cross-linked polyacrylic acid, polyvinyls or crosslinked polyvinyls, or polyacrylat and its copolymers.

The cross-linked polysaccharide is optionally and preferably at least one selected from the group consisting of insoluble metal salts or cross-linked derivatives of alginate, pectin, xantham gum, guar gum, tragacanth gum, and locust bean gum, carrageenan, metal salts thereof, and covalently cross-linked derivatives thereof.

The modified cellulose is optionally and preferably at least one selected from the group consisting of cross-linked derivatives of hydroxypropylcellulose, hydroxypropylmethylcellulose, hydroxyethylcellulose, methylcellulose, carboxymethylcellulose, and metal salts of carboxymethylcellulose.

Most preferably, the stress absorber is microcrystalline cellulose.

Optionally and preferably, the stress absorber at least partially coats each of the units.

The stress absorber can optionally be the sole excipient in the outer coating.

Alternatively, the outer coating may comprise an additional excipient.

The additional excipient is optionally and preferably at least one of a binder, a filler, a disintegrant, and an effervescent.

The binder is optionally and preferably at least one of Povidone (PVP: polyvinyl pyrrolidone), low molecular weight HPC (hydroxypropyl cellulose), low molecular weight HPMC (hydroxypropyl methylcellulose), carboxy methyl cellulose, hydroxylethyl cellulose, ethylcellulose, gelatin polyethylene oxide, acacia, dextrin, magnesium aluminum silicate, starch, and polymethacrylates.

The filler is optionally and preferably at least one of sugars such as lactose, glucose, fructose, or sucrose; dicalcium phosphate; sugar alcohols such as sorbitol, mannitol, maltitol, lactitol, xylitol, isomalt, erythritol, and hydrogenated starch hydrolysates; corn starch, potato starch, sodium carboxymethylcellulose, ethylcellulose and cellulose acetate, or a mixture thereof.

The disintegrant is optionally and preferably at least one of low-substituted carboxymethyl cellulose sodium, cross-linked polyvinyl pyrrolidone, sodium starch glycolate, cross-linked sodium carboxymethyl cellulose, pregelatinized starch, microcrystalline starch, water insoluble starch, calcium carboxymethyl cellulose, and low substituted hydroxypropyl cellulose magnesium aluminum silicate.

The outer coating may optionally further comprise at least one of a sweetener, a flavorant, a colorant, and a lubricant and an alkalizing agent.

According to further features in any of the embodiments of the invention, the benzimidazole is optionally and preferably at least one of omeprazole, lansoprazole and pantoprazole. More preferably, the benzimidazole is lansoprazole.

The benzimidazole may optionally comprise benzimidazole base. Alternatively, the benzimidazole may comprise a benzimidazole salt, such as, for example, the magnesium or sodium salt of omeprazole, or the sodium sesquihydrate of pantoprazole.

According to further features in any of the embodiments of the invention, the substrate optionally and preferably comprises a neutral core and an active coating containing the benzimidazole, the active coating being layered over the neutral core. The neutral core may comprise, for example, at least one of a non- pareil, a bead, a seed, a granule, or a pellet.

The non-pareil is optionally and preferably in the range of from about 80 to about 850 microns. More preferably, the non-pareil is in the range of from about 200 to about 250 microns.

Optionally and preferably, the substrate comprises an aqueous solvent.

According to further features in any of the embodiments of the invention, the enteric coating optionally and preferably comprises at least one enteric material selected from the group consisting of hydroxypropyl methylcellulose acetate succinate (hypromellose acetate succinate), cellulose acetate phthalate, hydroxypropyl methyl cellulose phthalate, polyvinyl acetate phthalate, sodium alginate, alginic acid, poly(methacrylic acid, methyl methacrylate)1:1 and (Eudragit L100), poly(methacrylic acid, ethyl acrylate)1:1 (Eudragit L30D-55).

Optionally and preferably, the enteric coating further comprises an organic solvent. More preferably, the enteric solvent comprises acetone. The enteric coating may optionally further comprise at least one excipient, such as, for example, a glidant, lubricant and anti-adherents, including but not limited to talc or titanium dioxide.

According to further features in any of the embodiments of the invention, each of the units optionally and preferably further comprises a sub-coating layered between the substrate and the enteric coating.

The sub-coating optionally and preferably further comprises an aqueous solvent.

Optionally and preferably, one or more of the substrate, and the sub-coating may further comprise an excipient.

The excipient may be at least one of a binder, a surfactant, a filler, a solubilizer, and an alkalizing agent.

Examples of binders include water-soluble, hydrophilic polymers, such as, for example, Povidone (PVP: polyvinyl pyrrolidone), low molecular weight HPC (hydroxypropyl cellulose), low molecular weight HPMC (hydroxypropyl methylcellulose), low molecular weight carboxy methyl cellulose, ethylcellulose, gelatin polyethylene oxide, acacia, dextrin, magnesium aluminum silicate, starch, and polymethacrylates, or a mixture thereof.

More preferably, the binder is hydroxypropyl methylcellulose (HPMC).

Examples of surfactants include polysorbate 80 (Tween 80) and sodium lauryl sulfate.

Examples of fillers include, for example, a sugar, such as lactose, glucose, fructose, or sucrose; dicalcium phosphate; sugar alcohols such as sorbitol, mannitol, maltitol, lactitol, xylitol, isomalt, erythritol, and hydrogenated starch hydrolysates ; corn starch; potato starch; sodium carboxymethylcellulose, ethylcellulose and cellulose acetate, or a mixture thereof.

More preferably, the filler is lactose.

Examples of alkalizing agents include sodium stearate, meglumine, disodium phosphate, and ammonia.

More preferably, the alkalizing agent comprises sodium stearate or meglumine.

Further embodiments of the present invent ion are laid out in the dependent claims.

The term "substrate" refers to substantially any structure which features the benzimidazole derivative, such as lansoprazole.

By "compressed units" it is meant units which have been subjected to sufficient compressional force to form a firm, cohesive tablet.

The phrase "stress absorber" refers to a material which is able to absorb a force applied to the outer coat, thereby preventing the force from being exerted on the enteric coat, and thus protecting the integrity of the enteric layer. This can be achieved by including in the outer coat a polymer having a suitable level of plasticity, or an appropriate particle structure and texture, or both.

When a polymer is exposed to a compressive load which causes the polymer to undergo deformation, the polymer behaves mechanically according to its stress/strain curve which is a mechanical finger print for that specific polymer. Accordingly, the mechanical behavior may be divided into two distinct regions: an elastic region (elastic deformation), and a plastic region (plastic deformation).

If the load is such that stress falls in the elastic region, then according to Hooke's law the strain will be proportional to stress (this proportionality can be expressed by a constant called Young's modulus). In such a case, if the load is removed the polymer can revert back to its original dimensions (a process known as elastic recovery, which is the percent of strain recovered when the load is released). Such a deformation is called elastic deformation. Elastic recovery increases with cross-linking, and may decrease as strain or stress increases.

If the material is stretched too far, Hooke's law ceases to hold and there will be permanent deformation known as plastic deformation. The point at which the stress-strain relationship departs from linear is called the "proportional or elastic limit" (yield point). Once the material has been stressed beyond the proportional limit, a permanent strain is present, even when the stress is reduced to zero. This process is called creep. Elastic materials generally have a larger region of linearity, and therefore a higher yield point. Deformation after the Proportional Limit contains recoverable strain (elastic), and non recoverable strain (plastic). The latter occurs as a result of the creep, and the process is accompanied by release of heat (energy loss).

The deformed stress absorber is energy rich, and this energy is released when the stress absorber is exposed to water. Without wishing to be limited by a single hypothesis, it is believed that the polymer or polymer mixture of the outer coating according to the present invention experiences both types of deformation. The plastic deformation causes the units to bind together as the polymer or polymer mixture of the outer coating on a unit may literally be pushed into that of another unit. However (and without wishing to be limited by a single hypothesis), at other points on the unit, the polymer or polymer mixture of the outer coating may undergo elastic deformation, which may absorb the stress and thereby preventing it from being exerted on the enteric coat, thereby preserving the integrity of the outer coating. The outer coating may optionally provide an additional layer of protection, for example against the entry of moisture through the outer coating and to the enteric coating which may occur during storage for example.

The phrase "enteric coating" refers to a layer which provides protection of the active ingredient against the acid environment of the stomach.

Hereinafter, the term "alkalizing agent" includes any material which is capable of providing a pH value of at least about 7.0 when present alone in water, preferably at least about 7.5 and more preferably at least about 8.0.

By "rapidly orally disintegratable" is meant that the tablet disintegrates upon oral administration, either in the mouth or upon swallowing, preferably prior to reaching the gastrointestinal tract. Disintegration would generally (optionally and preferably) occur within 60 seconds of administration of the tablet.

As used herein the term "about" refers to ± 10 %.

### DESCRIPTION OF THE PREFERRED EMBODIMENTS

The present invention is of a multiple unit tablet formulation for a benzimidazole, which is rapidly disintegratable upon oral administration, and can be formulated without the use of a plasticizer in the enteric coating.

The principles and operation of the compositions and methods according to the present invention may be better understood with reference to the accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

The present invention provides a rapidly orally or extra-orally disintegratable multiple unit tablet composition for a benzimidazole. The tablet comprises a multiplicity of units, consisting of a number of small, individual particles, which are compressed into a cohesive tablet. Compression involves subjecting the multiplicity of units, such as at least two individual units, to a crushing force.

Each unit comprises a substrate comprising a benzimidazole; an enteric coating, which is devoid of a plasticizer, layered on the substrate; and at least one type of an outer coating layered on the enteric coating, wherein said outer coating comprises a stress absorber.

The present invention also provides a method for producing a rapidly orally or extra-orally disintegratable composition, which method comprises providing a multiplicity of units, each unit comprising a substrate comprising benzimidazole, on which is layered an enteric coating being devoid of a plasticizer, and at least one type of an outer coating layered on the enteric coating, wherein said outer coating comprises a stress absorber; forming a mixture of the units; and compressing the mixture to form a tablet.

The composition of the present invention comprises as active ingredient a benzimidazole, such as omeprazole, lansoprazole, or pantoprazole, optionally in the form of a base. Alternatively, the benzimidazole may comprise a single enantiomer of a benzimidazole, or an alkaline benzimidazole salt, such as, for example, the magnesium or sodium salt of omeprazole, or the sodium sesquihydrate of pantoprazole, or one of its single enantiomers. Most preferably, the benzimidazole is lansoprazole, or a salt thereof.
Further aspects and embodiments of the present invention are laid out in the dependent claims.

### Substrate

The term "substrate" refers to substantially any structure which features the benzimidazole derivative, such as lansoprazole. For example, this structure could be an active core containing the benzimidazole derivative. This active core could be prepared in a number of different ways which are known in the art. For example, the active core could be formed by compressing the benzimidazole derivative with an alkaline substance. As another example, the active core could be prepared by mixing the benzimidazole derivative with an alkaline substance, spheronizing the mixture and then forming cores through pelletisation. As yet another example, the active core is optionally and preferably prepared by embedding the active ingredient in a poloxamer and compressing the embedded material into tablets. The active core is also optionally formed by granulating the active ingredient with an alkaline substance and compressing the granulation into tablets.

Alternatively and optionally and preferably, the structure could include a neutral core, such as a sugar bead which does not contain the benzimidazole derivative, over which the benzimidazole derivative is coated. The coating includes lansoprazole or other benzimidazole derivative with a suitable adhesive polymer. The neutral core may optionally comprise at least one of a non-pareil, a bead, a seed, a granule, and a pellet. Preferably, the core comprises a non-pareil or a pellet. The pellet optionally and preferably comprises microcrystalline cellulose. More preferably, the non-pareil has a size in the range of from about 80 to about 850 microns, most preferably in the range of from about 200 to about 250 microns. The coating on the substrate optionally further comprises an aqueous solvent.

The substrate may optionally comprise at least one excipient, such as a binder, a surfactant, and a filler.

Examples of suitable binders include but are not limited to water-soluble, hydrophilic polymers, such as Povidone (PVP: polyvinyl pyrrolidone), low molecular weight HPC (hydroxypropyl cellulose), low molecular weight HPMC (hydroxypropyl methylcellulose), low molecular weight carboxy methyl cellulose, hydroxyethylcellulose, gelatin, polyethylene oxide, acacia, dextrin, magnesium aluminum silicate, starch, and polymethacrylates. Optionally and preferably, the binder comprises hydroxypropyl methylcellulose or povidone.

Examples of suitable fillers include but are not limited to lactose, glucose, fructose, sucrose, dicalcium phosphate, sugar alcohols also known as "sugar polyol" such as sorbitol, mannitol, maltitol, lactitol, xylitol, isomalt, erythritol, and hydrogenated starch hydrolysates (a blend of several sugar alcohols), corn starch, potato starch, sodium carboxymethylcellulose, ethylcellulose and cellulose acetate, or a mixture thereof. Optionally and preferably, the filler comprises lactose.

Examples of suitable surfactants include but are not limited to polysorbate 80 (for example Tween 80, or sodium lauryl sulfate.

The substrate may optionally comprise an alkalizing agent, such as, for example, an inorganic basic salt, such as basic inorganic salts of sodium, magnesium or calcium, (such as sodium hydrogen carbonate, sodium stearate, disodium phosphate), meglumine, or ammonia. Examples of such basic inorganic salts of magnesium include, but are not limited to, heavy magnesium carbonate, magnesium carbonate, magnesium oxide, magnesium hydroxide, magnesium metasilicate aluminate, magnesium silicate aluminate, magnesium silicate, magnesium aluminate, synthetic hydrotalcite [Mg₆Al₂(OH)₁₆·CO₃·4H₂O] and aluminum magnesium hydroxide [2.5MgO·AhO₃·xH₂O]. Examples of such basic inorganic salts of calcium include, but are not limited to, precipitated calcium carbonate and calcium hydroxide.

Optionally and preferably, the alkalizing agent comprises sodium stearate or meglumine.

### Subcoating

Optionally and preferably, a formulation may feature a subcoating layer between the substrate and the enteric coating layer. The sub-coating layer is provided in order to prevent interaction between the enteric coating layer and the substrate containing the benzimidazole, particularly in embodiments wherein the substrate includes an alkalizing agent. The benzimidazole-containing alkaline reacting substrate is preferably separated from the enteric coating polymer(s) containing free carboxyl groups, which otherwise causes degradation/discoloration of the benzimidazole during the coating process or during storage.

The subcoating layer may optionally comprise at least one excipient, such as a binder, a surfactant, and a filler.

Examples of suitable binders include but are no limited to water-soluble, hydrophilic polymers, such as Povidone (PVP: polyvinyl pyrrolidone), low molecular weight HPC (hydroxypropyl cellulose) low molecular weight HPMC (hydroxypropyl methylcellulose), low molecular weight carboxy methyl cellulose, ethylcellulose, gelatin polyethylene oxide, acacia, dextrin, magnesium aluminum silicate, starch, and polymethacrylates. Optionally and preferably, the binder comprises HPMC.

Examples of suitable fillers include but are not limited to lactose, glucose sucrose, sorbitol, dicalcium phosphate, mannitol, maltitol, corn starch, potato starch, sodium carboxymethylcellulose, ethylcellulose and cellulose acetate. Optionally and preferably, the filler comprises lactose.

Examples of suitable surfactants include but are not limited to polysorbate 80 (for example Tween 80 or sodium lauryl sulfate). Optionally and preferably, the surfactant comprises polysorbate 80.

Alternatively, the subcoating layer may comprise Opadry II HP, based on polyvinyl alcohol.

The subcoating layer may optionally comprise an alkalizing agent, such as, for example, sodium stearate, meglumine, disodium phosphate, or ammonia. Optionally and preferably, the alkalizing agent is either sodium stearate or meglumine.

Examples of such basic inorganic salts of magnesium include, but are not limited to, heavy magnesium carbonate, magnesium carbonate, magnesium oxide, magnesium hydroxide, magnesium metasilicate aluminate, magnesium silicate aluminate, magnesium silicate, magnesium aluminate, synthetic hydrotalcite [Mg₆Al₂(OH)₁₆·CO₃·4H₂O] and aluminum magnesium hydroxide [2.5MgO·Al₂O₃·xH₂O]. Examples of such basic inorganic salts of calcium include, but are not limited to, precipitated calcium carbonate and calcium hydroxide.

### Enteric Coating

The formulation optionally and preferably features an enteric coating which is devoid of a plasticizer, the enteric coating comprises at least one enteric coating material. The enteric coating material is preferably a pH dependent polymer, more preferably a polymer selected from the group consisting of hydroxypropyl methylcellulose acetate succinate (also known as hypromellose acetate succinate), cellulose acetate phthalate, hydroxypropyl methyl cellulose phthalate, polyvinyl acetate phthalate, poly(methacrylic acid, methyl methacrylate)1:1 and poly(methacrylic acid, ethyl acrylate)1:1, alginic acid, and sodium alginate. A suitable enteric coating can be made from Eudragit™ polymers series (available from Rohm Pharma) which are polymeric lacquer substances based on acrylates and/or methacrylates. Suitable polymers which are slightly permeable to water, and exhibit a pH-dependent permeability include, but are not limited to, Eudragit™ S (poly(methacrylic acid, methyl methacrylate)1:2); Eudragit L100™ (poly(methacrylic acid, methyl methacrylate)1:1); Eudragit L30D™, (poly(methacrylic acid, ethyl acrylate)1:1); and (Eudragit L100-55) (poly(methacrylic acid, ethyl acrylate)1:1). Eudragit™ L is an anionic polymer synthesized from methacrylic acid and methacrylic acid methyl ester. It is insoluble in acids and pure water. It becomes soluble in neutral to weakly alkaline conditions. The permeability of Eudragit™ L is pH dependent. Above pH 5.0, the polymer becomes increasingly permeable. Mixtures of such polymers may also optionally be used.

Optionally and preferably, the enteric polymer comprises HPMC acetate succinate.

The enteric coating optionally further comprises an organic solvent, such as acetone. The enteric coating optionally further comprises an excipient, such as, for example, a glidant, such as talc or titanium dioxide.

### Outer Coating

The outer coating of the composition substantially entirely covers the enteric coating of each individual unit. This prevents direct contact between individual units in one hand and on the other hand absorbs the stress resulting from the compression force, all of which assists in protecting the integrity of the enteric coating of the units, increases flowability, prevents segregation, and provides excellent protection against humidity, thereby increasing the chemical stability of the benzimidazole. Additionally, the outer coating enables fast disintegration of the composition, while still permitting direct compression of the units of the composition into a tablet without requiring the addition of further tablet excipients.

The outer coating of the composition features a stress absorber. The stress absorber may be added to the outer coating prior to layering of the outer coating over the enteric coating layer.

The stress absorber may optionally and preferably at least partially coat the units of the composition.

As described in greater detail above, a stress absorber according to the teachings of the present invention is a material having a high degree of plasticity, such that the material can easily undergo deformation under stress, releasing the stress as heat, thereby enabling stress relaxation to occur during compression of the units of the composition to form a tablet. This prevents cracking of the enteric coating during the compression process.

The stress absorber according to any of the embodiments of the present invention may optionally comprise one of polysaccharides or cross-linked polysaccharides, starch, microcrystalline cellulose, ethyl cellulose, peptides or cross-linked peptides, protein or cross-linked proteins, gelatin or cross-linked gelatin, hydrolyzed gelatin or cross-linked hydrolyzed gelatin, collagen or cross-linked collagen, modified cellulose, polyacrylic acid or cross-linked polyacrylic acid, polyvinyls (such as polyvinylalcohol, polyvinyl acetate and polyvinyl pyrrolidone and their copolymers) or cross-linked polyvinyls, polyacrylat and its copolymers (such as Eudragit RL, Eudragit RS, Eudragit E, Eudragit L) or cross-linked polyacrylats. The cross-linked polysaccharide can be selected from the group consisting of insoluble metal salts or cross-linked derivatives of alginate, pectin, xantham gum, guar gum, tragacanth gum, and locust bean gum, carrageenan, metal salts thereof, and covalently cross-linked derivatives thereof. The modified cellulose may be selected from the group consisting of cross-linked derivatives of hydroxypropylcellulose, hydroxypropylmethylcellulose, hydroxyethylcellulose, methylcellulose, carboxymethylcellulose, and metal salts of carboxymethylcellulose.

Optionally and preferably, the stress absorber comprises microcrystalline cellulose.

The inclusion of a stress absorber in the outer coating prevents compression pressure from being exerted on the enteric coating. This abrogates the need for a plasticizer in the enteric coating, thereby avoiding the disadvantages associated with plasticizers, as discussed in greater detail in the Background section above. Hence, the enteric coating of a unit is devoid of a plasticizer.

The stress absorber may optionally be provided as the sole excipient in the outer coating.

Alternatively, the outer coating may comprise at least one further excipient, in addition to the stress absorber. Non-limiting examples of suitable excipients are described below. For example, the outer coating may optionally include a binder selected from the group including but not limited to a water-soluble hydrophilic polymer, such as Povidone (PVP: polyvinyl pyrrolidone), low molecular weight hydroxypropyl cellulose (HPC), low molecular weight hydroxypropyl methylcellulose (HPMC), low molecular weight carboxy methyl cellulose, hydroxyethylcellulose, gelatin, polyethylene oxide, acacia, dextrin, magnesium aluminum silicate, starch, and polymethacrylates.

Optionally and preferably, the concentration of the binder in the outer coating is in the range of from about 2 to about 15 %w/w of the total dry outer coating, and the concentration of stress absorber in the outer coating is in the range of from about 10 to about 50 %w/w of the total dry outer coating

The outer coating may optionally and preferably feature one or more fillers, optionally selected from the group including but not limited to sugars, such as lactose, glucose, fructose, sucrose, dicalcium phosphate, sugar alcohols such as sorbitol, mannitol, maltitol, lactitol, xylitol, isomalt, erythritol, and hydrogenated starch hydrolysates, corn starch, potato starch, sodium carboxymethycellulose, ethylcellulose and cellulose acetate, Pharmaburst® (a disintegrant based on mannitol) or a mixture thereof

Optionally and preferably, the concentration of the filler in the outer coating is in the range of from about 30 to about 70 %w/w of the total dry outer coating.

The outer coating may also optionally and preferably feature one or more disintegrants, optionally selected from the group including but not limited to low-substituted carboxymethyl cellulose sodium, crospovidone (cross-linked polyvinyl pyrrolidone), sodium carboxymethyl starch (sodium starch glycolate), cross-linked sodium carboxymethyl cellulose (Croscarmellose), pregelatinized starch (starch 1500), microcrystalline starch, water insoluble starch, calcium carboxymethyl cellulose, low substituted hydroxypropyl cellulose, and magnesium or aluminum silicate. The composition may optionally comprise at least two types of pellets, having the same substrate, subcoating layer and enteric coating, but differing in that a first of the two types of pellets includes a disintegrant in the outer coating, while a second of the two types of pellets is devoid of a disintegrant in the outer coating.

The mechanism of disintegration is based on swelling, wicking, and deformation of the disintegrants. Some commercial super-disintegrants for use in the present invention include but are not limited to Ac-Di-Sol, Primojel, Explotab, and Crospovidone.

The disintegrant preferably constitutes from about 2 to about 10 % of the total solid weight of the outermost layer.

The outer coating preferably features an effervescent. An effervescent material is optionally and preferably included in order to increase dissolution rate of the other excipients. The term "effervescent" includes compounds which evolve gas. The preferred effervescent agents evolve gas by means of a chemical reaction, such as between an acid and a base, which takes place upon exposure of the effervescent to water and other fluids. Such water-activated materials must be kept in a generally anhydrous state and with little or no absorbed moisture or in a stable hydrated form, since exposure to water will prematurely disintegrate the tablet.

The acid may be any which is safe for human consumption and may generally include but is not limited to food acids, acid and hydrite antacids including but not limited to, for example, citric, tartaric, malic, fumaric, adipic, and succinic.

The base may comprise a carbonate source. Carbonate sources include dry solid carbonate and bicarbonate salt such as, preferably, sodium bicarbonate, sodium carbonate, potassium bicarbonate and potassium carbonate, magnesium carbonate and the like. Carbonate sources such as sodium bicarbonate are preferable, in that they may also serve as an alkalizing agent. Optionally and preferably, the concentration of the effervescent in the outer coating is in the range of from about 5 to about 15 %w/w of the total dry outer coating.

The composition may optionally comprise at least two types of pellets, having the same substrate, subcoating layer and enteric coating, but differing in the composition of the outer coating, such that a first of the two portions includes an acid and a second of the two portions includes a base. For example, the outer coating of a first portion of pellets may comprise sodium carbonate and the outer coating of a second portion of pellets may comprise tartaric acid.

The outer coating may also optionally further comprise an additional excipient, such as one which increases palatability. Examples of additional excipients include a sweetener (such as acesulfame potassium), a flavorant (such as orange or mint flavor, or a combination thereof), a colorant, and a lubricant to ease in swallowing (such as polyethylene glycol). Flavorants and sweeteners are particularly useful when the active ingredient has a bitter taste, the masking of which would increase patient compliance.

The outer coating may optionally and preferably feature one or more alkalizing agents, optionally selected from the group including but not limited to sodium stearate, meglumine, disodium phosphate, and ammonia

The outer coating enables fast disintegration of the composition; allows direct compression of the units of the composition into a tablet without requiring the addition of further tablet excipients; and improves the stability of the pellets, which undergo discoloration in the absence of an outer coating layer. Fast or rapidly disintegrating means disintegration that occurs within not more than about 15 minutes.

### Preparation of formulations according to the present invention

The preparation of the compositions is described first with reference to the following general description and then with reference to the following non-limiting examples of the preparation and application of the compositions of the present invention.

As noted previously, the composition includes a substrate which features a benzimidazole. The substrate may be an active core containing the benzimidazole. This active core could be prepared in a number of different ways which are known in the art. For example, the active core could be formed by compressing benzimidazole with at least one excipient. As another example, the active core could be prepared by mixing the benzimidazole with the additional ingredient(s), spheronizing the mixture and then forming cores through pelletisation. The active core is also optionally formed by granulating the active ingredient with the additional ingredient(s) and compressing the granulation into tablets. The active core is also optionally formed by preparing pellets, and then compressing the pellets into a tablet.

The substrate is preferably prepared by dissolving the benzimidazole in an aqueous solvent, optionally also including at least one of a filler, a surfactant, a binder, a solubilizer and an alkalizing agent. This solution is then applied to an inert core, such as, for example, a non-pareil, a bead, a seed, a granule or a pellet.

The active layer may optionally be applied to the inert core by a dry coating process (dry powder layering), as discussed in detail below. Dry powder layering is particularly advantageous for application of the lansoprazole-containing active layer, since many excipients have been found to be incompatible with lansoprazole. This incompatibility is generally more apparant in a liquid medium such as a coating solution or suspension. In contrast, dry powder coating technology may provide a more stable manufacturing method for acid-labile drugs such as lansoprazole. For dry coating, lansoprazole can be applied to either sugar or microcrystalline spheres using an appropriate dry coating machine. The dusting powder, including the active material can be applied individually while spraying a binder solution. This dusting powder may further contain additional excipients, such as stabilizers, buffering agents, fillers, glidants, lubricants, surface active agents, solubilizers, dispersing agents, emulsifying agents, wetting agents, suspending agents, disintegrants, binders, and combinations thereof. Additionally, a second dusting powder can be applied to the pellets to enhance drug protection. This second dusting powder may contain fillers, disintegrants and binders.

Alternatively, the substrate may optionally be prepared without an inert core, by compression or wet granulation of these ingredients, or extrusion and spheronisation, or through any other suitable preparation method thereof.

Further alternatively, the substrate may be prepared by spheronization, such that the active material is encapsulated within a microsphere.

The subcoating layer is then coated over the substrate. Preferably, the subcoating layer is prepared by adding an organic basic salt, more preferably sodium stearate, as the alkaline agent, to an aqueous solution. Alternatively, the alkaline agent could be an inorganic basic salt as described below. The solution may also optionally include other ingredients, such as one or more surfactants, and/or one or more fillers, and/or one or more cellulosic polymers.

The subcoating layer can be applied to the substrate by conventional coating techniques such as, for instance, pan coating, fluidized bed coating, fluidized bed bottom sprayed coating or a Turbo Jet-Technology for the production of large amounts. Coating may be performed using a Fluidized Bed Processor (such as that of Glatt Gmbh), Unilab Fluidized Bed (Huttilin), or Ventilus Fluid Bed (Innojet). A fluidized bed is a bed of solid particles which are suspended in a stream of air or gas passing upward through the particles, in which the coating material is aerosolized. As the air travels through the particle bed, the particles are mixed in the stream of gas or air with the coating material, and so are coated but are also dried. Alternatively a turbo coating system may optionally be used.

Further alternatively, dry powder layering may be used for the subcoating layer. This process provides a number of advantages over conventional, liquid-based coating techniques. Dry powder layering has the advantage of enabling use of specific excipients while keeping their original properties. For example, it is well known that use of super-disintegrants or burst controlling agents in fast dissolving formulations may improve the disintegration rate. Using such materials in an aqueous-based coating formulation may, however, negatively alert their original properties, eventually causing a longer disintegration time. This problem may be totally overcome by use of a dry powder coating process.

A dry coating process also results in much lower energy requirements, more efficient utilization of coating materials, greater environmental friendliness, and lower operating costs, as compared to liquid-based methods. Pans used in aqueous coatings systems may be used for dry powder coatings processes, with only minor modifications. Since in dry powder coating, little or no solvent or water is used, it can be considered a more economical process than liquid coating processes, since vaporizing the liquid requires considerable energy consumption. Small dosage forms such as pellets and particles are currently coated in fluidized beds, which requires even larger amounts of hot air, and which may strengthen the concern about energy consumption when using a liquid-based or wet coating process. Use of organic solvents further results in environmental pollution, high solvent recycling costs, and danger of explosion during operation. Likewise, when using a wet coating process, air cleaning may also be a huge burden on the process, as the hot air has to be cleaned at both intake and outlet stages.

Dry powder coating processes may be carried out using many known systems, such as, for example, CF-Granulator (Freund Industrial, Tokyo, Japan), Granurex (Vector Corporation, Marion, IA, USA), GS HP/25 equipment (GS Coating System, Italy), Centrifugal Fluid Bed Granulator (Glatt, Germany) and other appropriate systems. A solution is then prepared with the enteric coating material. The solution preferably includes a solvent or a mixture thereof, including but not limited to, an aqueous solvent such as water, or an organic solvent such as isopropyl alcohol or other alcohols such as ethanol, or acetone. Mixtures of aqueous and organic solvents preferably include at least one polar organic solvent such as isopropyl alcohol for example. The solution may also optionally and preferably include a binder, and/or a surfactant.

This enteric coating solution is then layered over the previously coated (with the subcoating material) substrate to form the composition of the present invention. Any of the coating techniques described above for application of the subcoating layer may be used for layering of the enteric coating solution, including dry powder coating.

The outer coating layer is then layered over the enteric coating layer, again using any of the coating methods described above. Optionally, the enteric coated pellets may first be divided into at least two portions, and different coating layers applied to each of the two portions. For example, a first portion may be coated with a layer comprising an effervescent, while a second portion is coated with a layer devoid of an effervescent. The coating layers may further differ in other excipients, such as, for example, in flavorants.

If dry powder coating techniques are to be used for the outer coating layer, the enteric coated pellets may be placed in a coating system, and the dry powder applied while simultaneously spraying with a binder solution. A wide range of concentrations of binder solution may be used, with either aqueous or organic solvents. The binder solution may be continuously sprayed onto the moving pellets using a peristaltic pump. Addition of the powder may begin either at the same time, or shortly after, spraying of the binder solution begins. At the end of the process, the resultant over-coated pellets may be dried for an additional period of time prior to discharging, in order to allow any residues of the solvent to be vaporized as much as possible.

The rate, amount, homogeneity, inter- and intra-uniformity, efficiency, quality, and yield of the coating may be controlled by parameters such as batch size, rotor speed, binder spray rate, powder addition rate, inlet and outlet air temperature, bed temperature, atomization air pressure, air flap and air flow.

According to another example, the coating suspension can be prepared as follows. First hydroxypropyl cellulose (HPC) (8 g) was dissolved in purified water (600 g) to obtain a clear solution. Then PEG-2000 (6.7 g), sodium bicarbonate (14.1 g) and acesulfame potassium (1.3g) were added to the HPC solution and mixed to complete dissolution. Sorbitol (91.7 g), microcrystalline cellulose (49 g), crospovidone (9 g) and starch 1500 (5.5 g) were then added to the resulting clear solution and mixed with a Heidolph mixer to obtain a homogeneous suspension which was stirred throughout the coating process. The coating can be applied to the pellets by conventional coating techniques such as, for instance, pan coating, fluidized bed coating, fluidized bed bottom sprayed coating or a Turbo Jet-Technology for the production of large amounts. Coating may be performed using a Fluidized Bed Processor (such as that of Glatt Gmbh). A fluidized bed is a bed of solid particles which are suspended in a stream of air or gas passing upward through the particles, in which the coating material is aerosolized. As the air travels through the particle bed, the particles are mixed in the stream of gas or air with the coating material, and so are coated but are also dried. Alternatively a turbo coating system may optionally be used.

The coating solution or suspension may be based on dispersions in water and/or suitable organic solvents or by using latex suspensions of the polymers. Examples of enteric coating polymers are as given above.

The coated units are then compressed into a tablet, using any tabletting device known in the art. Compression is preferably performed at room temperature. "Room temperature" used herein refers to a temperature in a room at which compression is performed in manufacturing of the tablet, and the temperature is usually in the range of from about 20° C to about 23° C.

Compositions may, if desired, be presented in a pack or dispenser device, such as an FDA approved kit, which may contain one or more dosage forms containing the active ingredient. The pack may, for example, comprise metal or plastic foil, such as a blister pack. The pack or dispenser device may be accompanied by instructions for use.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above description, illustrate the invention in a non limiting fashion.

### Example 1

### A. Substrate

(i) Inert core: sugar beads (200-250 microns)
(ii) Active layer:

| **Ingredient** | **Function** | **Amount (%w/w of the total dry active layer weight)** |
|---|---|---|
| lansoprazole | active agent | 34.7 |
| HPMC | binder | 29.0 |
| polysorbate 80 | surfactant | 5.8 |
| lactose | filler | 29.0 |
| sodium stearate | alkalizing agent | 1.5 |
| water | solvent | Not present in final product |

HPMC is dissolved in purified water, until completely dissolved to form a first solution. A second solution is prepared by adding polysorbate 80, sodium stearate and lactose to purified water, until completely dissolved, after which lansoprazole is added to the solution. The first solution is then added to the second solution to form an active coating solution.

A fluidized bed coating device (Glatt, Germany) is loaded with the microcrystalline cellulose pellets. The active coating solution is sprayed on the pellets, using standard coating techniques, to form an active substrate.

Typical process parameters are: inlet temperature 50-55°C; automizing air pressure 0.8-1.3 bar; microclimate pressure 0.8-1.0 bar; product temperature 28-30°C; outlet air pressure 28-30°C.

### B. Subcoating layer

| **Ingredient** | **Function** | **Amount (%w/w of the total dry subcoating layer weight)** |
|---|---|---|
| HPMC | binder | 44.6 |
| polysorbate 80 | surfactant | 8.8 |
| lactose | filler | 44.6 |
| sodium stearate | alkalizing agent | 2 |
| water | solvent | Not present in final product |

The subcoating layer is applied to the active substrate, using standard spraying techniques, such as those described above.

### C. Enteric coating

| **Ingredient** | **Function** | **Amount (%w/w of the total dry enteric coating weight)** |
|---|---|---|
| HPMC acetate succinate | enteric material | 100 |
| acetone | solvent | Not present in final product |
| water | solvent | Not present in final product |

The enteric coating is applied over the subcoating layer, using standard spraying techniques, such as described in section A above.

### D. Outer coating

| **Ingredient** | **Function** | **Outer coating Amount (%w/w of the total dry outer coating weight)** |
|---|---|---|
| microcrystalline cellulose | stress absorber | 26.5 |
| sorbitol | filler | 49.5 |
| croscarmellose | disintegrant | 4.9 |
| Starch 1500 | disintegrant | 2.9 |
| HPC | binder | 4.3 |
| sodium bicarbonate | effervescent | 7.6 |
| acesulfame potassium | sweetener | 0.7 |
| PEG 2000 | lubricant | 3.6 |

The coating process was performed in a Fluidized bed Wurster coater (Uni-Glatt CN:6599) using one spray nozzle. The coating process was carried out under the following conditions: air inlet temperature 38-44°C, air outlet temperature 28-36°C, air pressure 2.5-2.6 bar and spray rate 6-12 ml/min.

The coated beads were compressed to 8 mm diameter tablets using a single punch tabletting machine (WICK). The hardness ranged between 12 kilo Newton (kN) and 20 kN.

For hardness of 12 kN, disintegration time was 1-3 minutes in buffer of pH 6.8 in a disintegration device, and 3-40 seconds in the mouth. For hardness of 18-20 kN, disintegration time was 3-5 minutes in buffer, and 1-1.5 minutes in the mouth.

### Example 2

The active layer, subcoating layer, and enteric layer were prepared and applied as for Example 1. The outer coating layer was prepared as follows:

| **Ingredient** | **Function** | **Formulation B Amount (%w/w of the total dry outer coating weight)** |
|---|---|---|
| microcrystalline cellulose | stress absorber | 28.5 |
| sorbitol | filler | 54.1 |
| HPC | binder | 4.6 |
| sodium bicarbonate | effervescent | 8.2 |
| acesulfame potassium | sweetener | 0.7 |
| PEG 2000 | lubricant | 3.9 |

### Example 3

The active layer, subcoating layer, and enteric layer were prepared and applied as for Example 1. The enteric coated pellets were then divided into two portions. A first portion was coated with outer coating A, and a second portion was coated with outer coating B.
As for Example A, except that outer coatings A and B are prepared as follows:

| **Ingredient** | **Function** | **Outer coating A Amount (%w/w of the total dry outer coating weight)** | **Outer coating B Amount (%w/w of the total dry outer coating weight)** |
|---|---|---|---|
| micro crystalline cellulose | stress absorber | 20.5 | 20.7 |
| Pharmaburst | filler | 40.8 | 41.4 |
| povidone | binder | 3.3 | 3.3 |
| sodium bicarbonate | effervescent | 30.0 | -- |
| acesulfame potassium | sweetener | 2.7 | 2.0 |
| PEG 2000 | lubricant | 2.7 | 2.8 |
| Tartaric acid | effervescent | -- | 27.0 |
| Orange flavor | Flavoring agent | -- | 2.8 |
| water | solvent | Not present in final product | Not present in final product |

After applying the outer coating layers, the two types of coated pellets were mixed in a ratio of 1:1. The pellets were compressed in a tableting machine (Korsch XL-100) to 10.3 mm tablets.

The resulting tablets had the following characteristics:

| **Parameter** | |
|---|---|
| Tablet weight | 350 mg |
| Thickness | 3.75 mm |
| Friability | 0.9% |
| Disintegration | 2.05 min |

### Example 4

As for Example 3, except that coating was performed using Huttlin's Unilab fluidized bed equipment. Typical process parameters were: inlet temperature 50-55°C; atomizing air pressure 1.0-1.8 bar; microclimate pressure 0.2-0.4 bar; product temperature 30-35°C.

### Example 5

As for Example 3, except that coating was performed using Innojet's Ventilus Fluid bed. The process included atomizing air pressure of 0.8-1.4 and support pressure of 0.2-0.5.

### Example 6

Tablets were prepared with a super-disintegrant outer coating layer as follows:

### A. Substrate

(i) Inert core: microcrystalline cellulose pellets (average size 250 microns)
(ii) Active layer:

| **Ingredient** | **Function** | **Amount (%w/w of the total dry active layer weight)** |
|---|---|---|
| lansoprazole | active agent | 32.1% |
| HPMC | binder | 24.3% |
| lactose | filler | 24.3% |
| Polysorbate 80 | surfactant | 9.3% |
| meglumine | alkalizing agent | 10% |
| water | solvent | Not present in final product |
| Total | | 100% |

The active layer is applied to the inert core as described above for Example 2.

### B. Subcoating layer

| Ingredient | Function | **Amount (%w/w of the total dry subcoating layer weight)** |
|---|---|---|
| HPMC | binder | 44.6% |
| lactose | filler | 44.6% |
| polysorbate 80 | surfactant | 8.8% |
| meglumine | alkalizing agent | 2% |
| water | solvent | Not present in final product |
| Total | | 100% |

The subcoatmg layer is applied to the active substrate, using standard spraying techniques, such as described in section A above.

### C. Enteric coating

| **Ingredient** | **Function** | **Amount (%w/w of the total dry enteric coating weight)** |
|---|---|---|
| HPMC acetate succinate | enteric material | 100% |
| acetone | solvent | Not present in final product |
| water | solvent | Not present in final product |
| Total | | 100% |

The eneric coating is applied over the subcoating layer, using standard spraying techniques, such as described in section A above.

### D. Outer coating layer

| **Ingredient** | **Function** | **Amount (%w/w of the total dry enteric coating weight)** |
|---|---|---|
| povidone | binder | 5.9% |
| polyethylene glycol | lubricant | 3.7% |
| croscarmellose sodium | disintergrant | 5.9% |
| mannitol | filler | 42% |
| sorbitol | filler | 13.8% |
| microcrystalline cellulose | stress absorber | 26.1% |
| acesulfame potassium | sweetener | 2.6% |
| water | solvent | Not present in final product |
| | | 100% |

The enteric coated pellets were compressed in a tableting machine (Korsch XL-100) to 10.3 mm or 14.4 mm tablets.

The resulting tablets had the following characteristics:

| **Parameter** | |
|---|---|
| Tablet weight | 400 mg |
| Thickness | 4.45 mm |
| Friability | 5% |
| Disintegration | 1.45 min |

Dissolution of the pellets showed resistance in gastric fluids after 1 hour in HC10.1N.

Dissolution of the compressed tablets showed release of over 75% within 30 min from buffer change.

### Example 7

The active layer, subcoating layer, and enteric layer were prepared and applied as for Example 6. The outer coating layer was prepared as follows:

| **Ingredient** | **Function** | **Outer coating A Amount (%w/w of the total dry outer coating weight)** | **Outer coating B Amount (%w/w of the total dry outer coating weight)** |
|---|---|---|---|
| microcrystalline cellulose | stress absorber | 19.5 | 19.5 |
| mannitol | filler | 30.8 | 30.8 |
| sorbitol | filler | 10.3 | 10.3 |
| povidone | binder | 3.3 | 4.4 |
| sodium bicarbonate | effervescent | 30.0 | -- |
| acesulfame potassium | sweetener | 2.1 | 1.0 |
| PEG 2000 | lubricant | 2.7 | 2.7 |
| tartaric acid | effervescent | -- | 26.9 |
| orange flavor | flavoring agent | -- | 2.2 |
| mint flavor | flavoring agent | -- | 2.2 |
| water | solvent | Not present in final product | Not present in final product |

The enteric coated pellets were then divided into two portions. A first portion was coated with outer coating A, and a second portion was coated with outer coating B.

After applying the outer coating layers, the two types of coated pellets were mixed in a ratio of 1:1. The pellets were compressed to tablets of diameter 10.3 mm.

The resulting tablets had the following characteristics:

| **Parameter** | |
|---|---|
| Tablet weight | 362 mg |
| Thickness | 3.9 mm |
| Friability | 0.8% |
| Disintegration | 1.30 min |

The compressed tablets showed a release of over 75% of the active material within 30 min from the buffer change.

### Example 8: Bioavailability study

A randomized, pharmacokinetic pilot study is undertaken to evaluate the bioavailability of test formulations of lansoprazole. For the study, lansoprazole tablets are prepared according to any suitable example above. A clinical study studies the issue of bioavailability. This study compares the efficacy and pharmacokinetic parameters of a tablet according to the present invention, with a MUPS reference product which contains a regular dosage of lansoprazole. It is believed that tablets prepared according to the present invention will show bioequivalence to a commercially available lansoprazole MUPS tablet product.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. A composition comprising a rapidly orally or extra-orally disintegratable tablet, the tablet comprising a multiplicity of compressed units, wherein each of said units comprises:
a substrate comprising benzimidazole;
an enteric coating layered on said substrate; and
at least one type of outer coating layered on said enteric coating, wherein said enteric coating is devoid of a plasticizer and said outer coating comprising a stress absorber.

2. The composition of claim 1,
wherein said outer coating prevents direct contact between said units; or
wherein said outer coating provides protection of said enteric coating against humidity, thereby increasing the chemical stability of said benzimidazole; or
wherein said outer coating provides good flowability; or
wherein said outer coating protects the integrity of said enteric coating during compression.

3. The composition of any of claims 1 to 2, wherein said tablet is disintegratable in a medium selected from the group consisting of aqueous solution, water and saliva.

4. The composition of any of claims 1 to 3, further comprising a sub-coating layered between said substrate and said enteric coating.

5. A method for producing a rapidly orally or extra-orally disintegratable composition comprising:
(a) providing a multiplicity of units according to claim 1;
(b) forming a mixture of said multiplicity of units; and
(c) compressing said mixture to form a tablet.

6. The method of claim 5, wherein said substrate is produced by dissolving said benzimidazole in an aqueous dispersion and spraying said dispersion onto an inert core.

7. The method of claim 6, wherein said substrate is produced by a method selected from the group consisting of compression, granulation, extrusion and spheronization.

8. The composition of any of claims 1 to 4, wherein said outer coating comprises at least one of a binder, a filler, a disintegrant, and an effervescent.

9. The composition of claim 8, wherein said multiplicity of compressed units comprises a first portion of said units and a second portion of said units, wherein said outer coating of said first portion of said units comprises an acid and said second outer coating of said second portion of said units comprises a base, wherein said acid and said base comprise said effervescent.

10. The composition of claim 8, wherein said binder is selected from the group consisting of Povidone (PVP: polyvinyl pyrrolidone), low molecular weight HPC (hydroxypropyl cellulose), low molecular weight HPMC (hydroxypropyl methylcellulose), carboxy methyl cellulose, hydroxyethyl cellulose, ethylcellulose, gelatin polyethylene oxide, acacia, dextrin, magnesium aluminum silicate, starch, and polymethacrylates, and a mixture thereof.

11. The composition of claim 8, wherein said filler is selected from the group consisting of a sugar, dicalcium phosphate, a sugar alcohol, corn starch, potato starch, sodium carboxymethylcellulose, ethylcellulose and cellulose acetate, and a mixture thereof, preferably wherein said sugar is selected from the group consisting of lactose, glucose, fructose, and sucrose, or preferably wherein said sugar alcohol is selected from the group consisting of sorbitol, mannitol, maltitol, lactitol, xylitol, isomalt, erythritol, and hydrogenated starch hydrolysates.

12. The composition of claim 8, wherein said disintegrant is selected from the group consisting of low-substituted carboxymethyl cellulose sodium, cross-linked polyvinylpyrrolidone , sodium starch glycolate, cross-linked sodium carboxymethyl cellulose, pregelatinized starch, microcrystalline starch, water insoluble starch, calcium carboxymethyl cellulose, low substituted hydroxypropyl cellulose, magnesium aluminum silicate, and a mixture thereof.

13. The composition of any of claims 1 to 4, or 8 to 12, wherein said outer coating further comprises at least one of a sweetener, a flavorant, a colorant, a lubricant and an alkalizing agent, preferably wherein said sweetener comprises acesulfame potassium, or preferably wherein said flavorant is selected from the group consisting of orange flavor, mint flavor, and a combination thereof.

14. The composition of claim 8,
wherein said effervescent is selected from the group consisting of food acids, acid and hydrite antacids, or
wherein said effervescent is selected from the group consisting of citric, tartaric, malic, fumaric, adipic, and succinic acids, or
wherein said effervescent is a carbonate, preferably wherein said carbonate is selected from the group consisting of sodium bicarbonate, sodium carbonate, potassium bicarbonate and potassium carbonate, and magnesium carbonate, or
wherein said effervescent comprises an acid and a base.

15. The composition of any of claims 1 to 4, or 8 to 14, wherein said benzimidazole is selected from the group consisting of omeprazole, lansoprazole and pantoprazole, preferably wherein said benzimidazole is lansoprazole.

16. The composition of claim 15,
wherein said benzimidazole comprises a benzimidazole base, or
wherein said benzimidazole comprises a benzimidazole salt.

17. The composition of claim 16,
wherein said benzimidazole comprises omeprazole and said salt is a magnesium or sodium salt, or
wherein said benzimidazole comprises pantoprazole and said salt is a sodium sesquihydrate.

18. The composition of any of claims 1 to 4 or 8 to 17, wherein said substrate comprises a neutral core and an active coating containing the benzimidazole, said active coating being layered over said neutral core.

19. The composition of claim 18, wherein said neutral core comprises a non- pareil, a bead, a seed, a granule, or a pellet, preferably wherein said neutral core is a microcrystalline cellulose pellet, or preferably wherein said non-pareil has a size in the range of from about 80 to about 850 microns, more preferably wherein said non-pareil has a size in the range of from about 200 to about 250 microns.

20. The composition of any of claims 1 to 4 or 8 to 19, wherein said substrate comprises an aqueous solvent.

21. The composition of any of claims 1 to 4 or 8 to 20, wherein said enteric coating comprises at least one enteric material selected from the group consisting of hydroxypropyl methylcellulose acetate succinate (hypromellose acetate succinate), cellulose acetate phthalate, hydroxypropyl methyl cellulose phthalate, polyvinyl acetate phthalate, alginic acid, and sodium alginate, Eudragit S™; Eudragit L 100™; Eudragit L30D™; Eudragit L100- 55 and Eudragit™ L and mixtures thereof, preferably wherein said enteric coating further comprises a glidant,
or preferably wherein said enteric coating further comprises an organic solvent, more preferably wherein said organic solvent is acetone.

22. The composition of claim 4,
wherein said sub-coating further comprises an aqueous solvent, or
wherein said sub-coating comprises Opadry II HP.

23. The composition of any of claims 1 to 4 or 8 to 22, wherein said substrate further comprises at least one of a binder, a surfactant, a filler, a solubilizer, and an alkalizing agent.

24. The composition of claim 23, wherein said binder comprises a water- soluble, hydrophilic polymer,
preferably wherein said polymer is selected from the group consisting of Povidone (PVP: polyvinyl pyrrolidone), low molecular weight HPC (hydroxypropyl cellulose), low molecular weight HPMC (hydroxypropyl methylcellulose), low molecular weight carboxy methyl cellulose, ethylcellulose, gelatin polyethylene oxide, acacia, dextrin, magnesium aluminum silicate, starch, and polymethacrylates, and a mixture thereof, more preferably wherein said binder is hydroxypropyl methylcellulose (HPMC).

25. The composition of claim 23, wherein said surfactant comprises polysorbate 80 (Tween 80).

26. The composition of claim 23, wherein said filler is selected from the group consisting of a sugar; dicalcium phosphate; a sugar alcohol; corn starch, potato starch, sodiumcarboxymethylcellulose, ethylcellulose and cellulose acetate, and a mixture thereof,
preferably wherein said sugar is selected from the group consisting of lactose, glucose, fructose, and sucrose, more preferably wherein said filler is lactose, or
preferably wherein said sugar alcohol is selected from the group consisting of sorbitol, mannitol, maltitol, lactitol, xylitol, isomalt, erythritol, and hydrogenated starch hydrolysate.

27. The composition of claim 23, wherein said alkalizing agent is selected from the group consisting of sodium stearate, meglumine, disodium phosphate and ammonia,
preferably wherein said alkalizing agent is sodium stearate, or
preferably wherein said alkalizing agent is meglumine.

28. The composition of claim 1, wherein at least a portion of said multiplicity of compressed units have separated upon entering the gastro-intenstinal tract.

29. The composition of any of claims 1 to 4 or 8 to 28, wherein said stress absorber is selected from the group consisting of polysaccharides or cross-linked polysaccharides, starch, microcrystalline cellulose, ethyl cellulose, peptides or cross-linked peptides, protein or cross-linked proteins, gelatin or cross-linked gelatin, hydrolyzed gelatin or cross-linked hydrolyzed gelatin, collagen or cross-linked collagen, modified cellulose, polyacrylic acid or cross-linked polyacrylic acid, polyvinyls or cross linked polyvinyls, polyacrylat and its copolymers, and mixtures thereof,
preferably
wherein said cross-linked polysaccharide is selected from the group consisting of insoluble metal salts or cross-linked derivatives of alginate, pectin, xanthan gum, guar gum, tragacanth gum, and locust bean gum, carrageenan, metal salts thereof, and covalently cross-linked derivatives thereof, or
wherein said modified cellulose is selected from the group consisting of cross-linked derivatives of hydroxypropylcellulose, hydroxypropylmethylcellulose, hydroxyethylcellulose, methylcellulose, carboxymethylcellulose, and metal salts of carboxymethylcellulose, and mixtures thereof, or
wherein said stress absorber is microcrystalline cellulose.

## Patentansprüche

1. Zusammensetzung, welche eine oral oder extra-oral schnell zersetzbare Tablette umfasst, worin die Tablette eine Vielzahl komprimierter Einheiten umfasst, worin jede der Einheiten umfasst:
ein Benzimidazol enthaltendes Substrat;
eine magensaftresistente, auf das Substrat aufgebrachte Schicht; und
mindestens einen Typ einer äußeren Schicht, die auf die magensaftresistente Schicht aufgebracht wurde,
worin die magensaftresistente Schicht keinen Weichmacher und die äußere Schicht ein Stress-Absorptionsmittel enthält.

2. Zusammensetzung nach Anspruch1,
worin die äußere Schicht einen direkten Kontakt zwischen den Einheiten verhindert; oder
worin die äußere Schicht Schutz für die magensaftresistente Schicht gegen Feuchtigkeit liefert, wodurch die chemische Stabilität des Benzimidazols erhöht wird; oder
worin die äußere Schicht eine gute Rieselfahigkeit liefert; oder
worin die äußere Schicht die Integrität der magensaftresistenten Schicht während der Kompression schützt.

3. Zusammensetzung nach einem der Ansprüche 1 bis 2, worin die Tablette in einem Medium nicht zersetzbar ist, ausgewählt aus der Gruppe bestehend aus wäßriger Lösung, Wasser und Speichel.

4. Zusammensetzung nach einem der Ansprüche 1 bis 3, welche weiter eine Unterschicht umfasst, die zwischen dem Substrat und der magensaftresistenten Schicht vorgesehen ist.

5. Verfahren zur Herstellung einer oral oder extra-oral schnell zersetzbaren Tablette, welche umfasst:
(a) Bereitstellen einer Vielzahl von Einheiten gemäß Anspruch 1;
(b) Bilden eines Gemisches der Vielzahl von Einheiten; und
(c) Komprimieren des Gemisches, um eine Tablette zu bilden.

6. Verfahren nach Anspruch 5, worin das Substrat durch Lösen von Benzimidazol in einer wäßrigen Dispersion und Sprühen der Dispersion auf einen inerten Kern hergestellt wird.

7. Verfahren nach Anspruch 6, worin das Substrat durch ein Verfahren hergestellt wird ausgewählt aus der Gruppe bestehend aus Kompression, Granulierung, Extrusion und Kügelchen-Bildung.

8. Zusammensetzung nach einem der Ansprüche 1 bis 4, worin die äußere Schicht mindestens ein Bindemittel, ein Füllmittels, ein Zersetzungsmittels, und ein Schäummittel umfasst.

9. Zusammensetzung nach Anspruch 8, worin die Vielzahl komprimierter Einheiten einen ersten Bereich der Einheiten und einen zweiten Bereich der Einheiten umfasst, worin die äußere Schicht des ersten Bereichs der Einheiten eine Säure umfasst und die zweite äußere Schicht des zweiten Bereichs der Einheiten eine Base umfasst, worin die Säure und die Base ein Schaummittel umfassen.

10. Zusammensetzung nach Anspruch 8, worin das Bindemittel ausgewählt ist aus der Gruppe bestehend aus Povidon (PVP: Polyvinylpyrrolidon), HPC (Hydroxypropylcellulose) mit niedrigem Molekulargewicht, HPMC (Hydroxypropylmethylcellulose) mit niedrigem Molekulargewicht, Carboxymethylcellulose, Hydroxyethylcellulose, Ethylcellulose, Gelatinpolyethylenoxid, Acacia, Dextrin, Magnesiumaluminumsilikat, Stärke und Polymethacrylaten, und einem Gemisch davon.

11. Zusammensetzung nach Anspruch 8, worin das Füllmittel ausgewählt ist aus der Gruppe bestehend aus einem Zucker, Dicalciumphosphat, einem Zuckeralkohol, Kornstärke, Kartoffelstärke, Natruimcarboxymethylcellulose, Ethylcellulose und Celluloseacetat, und einem Gemisch davon, vorzugsweise worin der Zucker ausgewählt ist aus der Gruppe bestehend aus Lactose, Glucose, Fructose und Saccharose, oder vorzugsweise worin der Zuckeralkohol ausgewählt ist aus der Gruppe bestehend aus Sorbit, Mannitol, Maltitol, Lactitol, Xylitol, Isomalt, Erythritol und hydrierten Stärkehydrolysaten.

12. Zusammensetzung nach Anspruch 8, worin das Zersetzungsmittel ausgewählt ist aus der Gruppe bestehend aus gering-substitutierter Carboxymethylcellulose-Natrium, vernetztem Polyvinylpyrrolidon, Natrium-Stärkeglycolat, vernetzter Natriumcarboxymethylcellulose, vorgelatinisierter Stärke, mikrokrystalliner Stärke, Wasser-unlöslicher Stärke, Calciumcarboxymethylcellulose, gering-substituierter Hydroxypropylcellulose, Magnesiumaluminumsilikat und einem Gemisch davon.

13. Zusammensetzung nach einem der Ansprüche 1 bis 4, oder 8 bis 12, worin die äußere Schicht weiter mindestens einen Süßstoff, einen Geschmacksstoff, ein Färbemittel, ein Schmiermittel und ein alkalisierendes Mittel umfasst, vorzugsweise
worin der Süßstoff Acesulfamkalium umfasst, oder vorzugsweise
worin der Geschmacksstoff ausgewählt ist aus der Gruppe bestehend aus Orangengeschmack, Minzgeschmack und einer Kombination davon.

14. Zusammensetzung nach Anspruch 8,
worin das Schäummittel ausgewählt ist aus der Gruppe bestehend aus Nahrungsmittelsäuren, Säure- und Hydrit-Antaciden, oder
worin das Schäummittel ausgewählt ist aus der Gruppe bestehend aus Zitronensäure, Weinsäure, Maleinsäure, Fumarsäure, Adipinsäure und Succinsäure, oder
worin das Schäummittel ein Carbonat ist, vorzugsweise worin das Carbonat ausgewählt ist aus der Gruppe bestehend aus Natriumbicarbonat, Natriumcarbonat, Kaliumbicarbonat und Kaliumcarbonat und Magnesiumcarbonat, oder
worin das Schäummittel eine Säure und eine Base umfasst.

15. Zusammensetzung nach einem der Ansprüche 1 bis 4, oder 8 bis 14, worin das Benzimidazol ausgewählt ist aus der Gruppe bestehend aus Omeprazol, Lansoprazol und Pantoprazol, vorzugsweise
worin das Benzimidazol is Lansoprazol ist.

16. Zusammensetzung nach Anspruch15, worin das Benzimidazol eine Benzimidazol-Base umfasst, oder
worin das Benzimidazol ein Benzimidazolsalz umfasst.

17. Zusammensetzung nach Anspruch16,
worin das Benzimidazol Omeprazol umfasst und das Salz ein Magnesium- oder Natrium-Salz ist, oder
worin das Benzimidazol Pantoprazol ist und das Salz ein Natrium-Sesquihydrat ist.

18. Zusammensetzung nach einem der Ansprüche 1 bis 4 oder 8 bis 17, worin das Substrat einen neutralen Kern umfasst und eine aktive Beschichtung, die das Benzimidazol enthält, worin die aktive Beschichtung über den neutralen Kern aufgetragen ist.

19. Zusammensetzung nach Anspruch 18, worin der neutral Kern ein Non-Pareil, ein Kügelchen, einen Keim, ein Granulat oder ein Pellet umfasst, vorzugsweise
worin der neutrale Kern ein mikrokristallines Cellulosepellet ist, oder vorzugsweise
worin der Non-Pareil eine Größe im Bereich von etwa 80 bis etwa 850 µm aufweist, mehr bevorzugt
worin das Non-Pareil eine Größe im Bereich von etwa 200 bis etwa 250 µm aufweist.

20. Zusammensetzung nach einem der Ansprüche 1 bis 4 oder 8 bis 19, worin das Substrat ein wäßriges Lösungsmittel umfasst.

21. Zusammensetzung nach einem der Ansprüche 1 bis 4 oder 8 bis 20, worin die magensaftresistente Schicht mindestens ein enterales Material umfasst ausgewählt aus der Gruppe bestehend aus Hydroxypropylmethylcelluloseacetatsuccinat (Hypromelloseacetatsuccinat), Celluloseacetatphthalat, Hydroxypropylmethylcellulosephthalat, Polyvinylacetatphthalat, Alginsäure und Natriumalginat, Eudragit STM; Eudragit L 100TM; Eudragit L30DTM; Eudragit L100- 55 und EudragitTM L und Gemische davon, vorzugsweise worin die magensaftresistente Schicht weiter ein Gleitmittel umfasst, oder vorzugsweise
worin die magensaftresistente Schicht weiter ein organisches Lösungsmittel umfasst, mehr bevorzugt
worin das organische Lösungsmittel Aceton ist.

22. Zusammensetzung nach Anspruch 4,
worin die Unterschicht weiter ein wäßriges Lösungsmittel umfasst, oder
worin die Unterschicht Opadry II HP umfasst.

23. Zusammensetzung nach einem der Ansprüche 1 bis 4 oder 8 bis 22, worin das Substrat weiter mindestens ein Bindemittel umfasst, ein oberflächenaktives Mittel, einen Füllstoff, einen Lösungsvermittler und ein alkalisierendes Mittel.

24. Zusammensetzung nach Anspruch 23, worin die Bindemittel ein Wasser-lösliches, hydrophiles Polymer umfasst, vorzugsweise
worin das Polymer ausgewählt ist aus der Gruppe bestehend aus Povidon (PVP: Polyvinylpyrrolidon), HPC (Hydroxypropylcellulose) mit niedrigem Molekulargewicht, HPMC (Hydroxypropylmethylcellulose) mit niedrigem Molekulargewicht, Carboxymethylcellulose mit niedrigem Molekulargewicht, Ethylcellulose, Gelatinpolyethylenoxid, Acacia, Dextrin, Magnesiumaluminumsilikat, Stärke und Polymethacrylaten und einem Gemisch davon, mehr bevorzugt
worin das Bindemittel Hydroxypropylmethylcellulose (HPMC) ist.

25. Zusammensetzung nach Anspruch 23, worin das oberflächenaktive Mittel Polysorbat 80 (Tween 80) umfasst.

26. Zusammensetzung nach Anspruch 23, worin der Füllstoff ausgewählt ist aus der Gruppe bestehend aus einem Zucker; Dicalciumphosphat; einem Zuckeralkohol; Kornstärke, Kartoffelstärke, Natriumcarboxymethylcellulose, Ethylcellulose und Celluloseacetat und einem Gemisch davon, vorzugsweise
worin der Zucker ausgewählt ist aus der Gruppe bestehend aus Lactose, Glucose, Fructose, und Saccharose, mehr bevorzugt worin der Füllstoff Lactose ist, oder vorzugsweise worin der Zuckeralkohol ausgewählt ist aus der Gruppe bestehend aus Sorbit, Mannitol, Maltitol, Lactitol, Xylitol, Isomalt, Erythritol, und hydriertem Stärkehydrolysat.

27. Zusammensetzung nach Anspruch 23, worin das alkalisierende Mittel ausgewählt ist aus der Gruppe bestehend aus Natriumstearat, Meglumin, Dinatriumphosphat und Ammonium, vorzugsweise worin das alkalisierende Mittel Natriumstearat ist oder vorzugsweise
worin das alkalisierende Mittel Meglumin ist.

28. Zusammensetzung nach Anspruch1, worin sich mindestens ein Teil der Vielzahl komprimierter Einheiten beim Eintritt in den Verdauungstrakt getrennt hat.

29. Zusammensetzung nach einem der Ansprüche 1 bis 4 oder 8 bis 28, worin das Stress-Absorptionsmittel ausgewählt ist aus der Gruppe bestehend aus Polysacchariden oder vernetzten Polysacchariden, Stärke, mikrokrystalliner Cellulose, Ethylcellulose, Peptiden oder vernetzten Peptiden, Protein oder vernetzten Proteinen, Gelatine oder vernetzter Gelatine, hydrolisierter Gelatine oder vernetzter hydrolisierter Gelatine, Kollagen oder vernetztem Kollagen, modifizierter Cellulose, Polyacrylsäure oder vernetzter Polyacrylsäure, Polyvinylen oder vernetztem Polyvinylen, Polyacrylat und dessen Copolymeren, und Gemischen davon, vorzugsweise
worin das vernetzte Polysaccharid ausgewählt ist aus der Gruppe bestehend aus unlöslichen Metallsalzen oder vernetzten Alginat-Derivaten, Pectin, Xanthangum, Guargum, Tragacanthgum und Locust Bean Gum, Carrageenan, Metallsalzen davon und kovalent vernetzten Derivaten davon, oder vorzugsweise
worin die modifizierte Cellulose ausgewählt ist aus der Gruppe bestehend aus vernetzten Derivaten von Hydroxypropylcellulose, Hydroxypropylmethylcellulose, Hydroxyethylcellulose, Methylcellulose, Carboxymethylcellulose und Metallsalzen von Carboxymethylcellulose und Gemischen davon, oder vorzugsweise
worin das Stress-Absorptionsmittel mikrokristalline Cellulose ist.

## Revendications

1. Composition comprenant un comprimé apte à se désintégrer rapidement par voie orale ou extra-orale, le comprimé comprenant une multiplicité d'unités comprimées, chacune desdites unités comprenant :
un substrat comprenant du benzimidazole ;
un revêtement gastro-résistant en couches sur ledit substrat ; et
au moins un type de revêtement externe en couches sur ledit revêtement gastro-résistant,
ledit revêtement gastro-résistant étant dépourvu de plastifiant et ledit revêtement externe comprenant un absorbeur de contrainte.

2. Composition selon la revendication 1,
ledit revêtement externe empêchant un contact direct entre lesdites unités ; ou
ledit revêtement externe assurant la protection dudit revêtement gastro-résistant vis-à-vis de l'humidité, ce qui permet d'augmenter la stabilité chimique dudit benzimidazole ; ou
ledit revêtement externe assurant une bonne aptitude à l'écoulement ; ou
ledit revêtement externe protégeant l'intégrité dudit revêtement gastro-résistant pendant la compression.

3. Composition selon l'une quelconque des revendications 1 à 2, ledit comprimé étant apte à se désintégrer dans un milieu sélectionné dans le groupe constitué par une solution aqueuse, l'eau et la salive.

4. Composition selon l'une quelconque des revendications 1 à 3, comprenant en outre un sous-revêtement en couches entre ledit substrat et ledit revêtement gastro-résistant.

5. Procédé de production d'une composition apte à se désintégrer rapidement par voie orale ou extra-orale comprenant :
(a) la mise à disposition d'une multiplicité d'unités selon la revendication 1 ;
(b) la formation d'un mélange de ladite multiplicité d'unités ; et
(c) la compression dudit mélange pour former un comprimé.

6. Procédé selon la revendication 5, ledit substrat étant produit par dissolution dudit benzimidazole dans une dispersion aqueuse et pulvérisation de ladite dispersion sur un noyau inerte.

7. Procédé selon la revendication 6, ledit substrat étant produit par un procédé sélectionné dans le groupe constitué par la compression, la granulation, l'extrusion et la sphéronisation.

8. Composition selon l'une quelconque des revendications 1 à 4, ledit revêtement externe comprenant au moins l'un parmi un liant, une charge, un délitant et un agent effervescent.

9. Composition selon la revendication 8, ladite multiplicité d'unités comprimées comprenant une première partie desdites unités et une seconde partie desdites unités, ledit revêtement externe de ladite première partie desdites unités comprenant un acide et ledit second revêtement externe de ladite seconde partie desdites unités comprenant une base, ledit acide et ladite base comprenant ledit agent effervescent.

10. Composition selon la revendication 8, ledit liant étant sélectionné dans le groupe constitué par la Povidone (PVP : polyvinyl pyrrolidone), l'HPC de faible poids moléculaire (hydroxypropyl cellulose), l'HPMC de faible poids moléculaire (hydroxypropyl méthylcellulose), la carboxy méthyl cellulose, l'hydroxyéthyl cellulose, l'éthylcellulose, la gélatine, l'oxyde de polyéthylène, l'acacia, la dextrine, le silicate d'aluminium et de magnésium, l'amidon et les polyméthacrylates, et un mélange de ceux-ci.

11. Composition selon la revendication 8, ladite charge étant sélectionnée dans le groupe constitué par un sucre, le phosphate dicalcique, un alcool de sucre, l'amidon de maïs, l'amidon de pomme de terre, la carboxyméthylcellulose de sodium, l'éthylcellulose et l'acétate de cellulose, et un mélange de ceux-ci, ledit sucre étant sélectionné de préférence dans le groupe constitué par le lactose, le glucose, le fructose et le saccharose, ou ledit alcool de sucre étant sélectionné de préférence dans le groupe constitué par le sorbitol, le mannitol, le maltitol, le lactitol, le xylitol, l'isomalt, l'érythritol et les hydrolysats d'amidon hydrogénés.

12. Composition selon la revendication 8, ledit délitant étant sélectionné dans le groupe constitué par le sodium de carboxyméthyl cellulose à faible degré de substitution, la polyvinylpyrrolidone réticulée, le glycolate d'amidon sodique, la carboxyméthyl cellulose de sodium réticulée, l'amidon prégélatinisé, l'amidon microcristallin, l'amidon insoluble dans l'eau, la carboxyméthyl cellulose de calcium, l'hydroxypropyl cellulose à faible degré de substitution, le silicate d'aluminium et de magnésium, et un mélange de ceux-ci.

13. Composition selon l'une quelconque des revendications 1 à 4, ou 8 à 12, ledit revêtement externe comprenant en outre au moins l'un parmi un édulcorant, un agent aromatisant, un colorant, un lubrifiant et un agent alcalinisant, ledit édulcorant comprenant de préférence du potassium d'acésulfame, ou ledit aromatisant étant sélectionné de préférence dans le groupe constitué par un arôme d'orange, un arôme de menthe, et une combinaison de ceux-ci.

14. Composition selon la revendication 8,
ledit agent effervescent étant sélectionné dans le groupe constitué par les acides alimentaires, les acides et les antiacides hydrites, ou
ledit agent effervescent étant sélectionné dans le groupe constitué par les acides citrique, tartrique, malique, fumarique, adipique et succinique, ou
ledit agent effervescent étant un carbonate, ledit carbonate étant sélectionné de préférence dans le groupe constitué par le bicarbonate de sodium, le carbonate de sodium, le bicarbonate de potassium et le carbonate de potassium, et le carbonate de magnésium, ou
ledit agent effervescent comprenant un acide et une base.

15. Composition selon l'une quelconque des revendications 1 à 4, ou 8 à 14, ledit benzimidazole étant sélectionné dans le groupe constitué par l'oméprazole, le lansoprazole et le pantoprazole, ledit benzimidazole étant de préférence le lansoprazole.

16. Composition selon la revendication 15,
ledit benzimidazole comprenant une base de benzimidazole, ou
ledit benzimidazole comprenant un sel de benzimidazole.

17. Composition selon la revendication 16,
ledit benzimidazole comprenant de l'oméprazole et ledit sel étant un sel de magnésium ou de sodium, ou
ledit benzimidazole comprenant du pantoprazole et ledit sel étant un sesquihydrate de sodium.

18. Composition selon l'une quelconque des revendications 1 à 4 ou 8 à 17, ledit substrat comprenant un noyau neutre et un revêtement actif contenant le benzimidazole, ledit revêtement actif étant en couches sur ledit noyau neutre.

19. Composition selon la revendication 18, ledit noyau neutre comprenant une nonpareille, une bille, une graine, un granulé ou une pastille, ledit noyau neutre étant de préférence une pastille de cellulose microcristalline, ou ladite nonpareille présentant de préférence une taille dans la plage d'environ 80 à environ 850 microns, ladite nonpareille présentant plus préférablement une taille dans la plage d'environ 200 à environ 250 microns.

20. Composition selon l'une quelconque des revendications 1 à 4 ou 8 à 19, ledit substrat comprenant un solvant aqueux.

21. Composition selon l'une quelconque des revendications 1 à 4 ou 8 à 20, ledit revêtement gastro-résistant comprenant au moins une matière gastro-résistante sélectionnée dans le groupe constitué par le succinate d'acétate d'hydroxypropyl méthylcellulose (succinate d'acétate d'hypromellose), le phtalate d'acétate de cellulose, le phtalate d'hydroxypropyl méthyl cellulose, le phtalate d'acétate de polyvinyle, l'acide alginique, et l'alginate de sodium, l'Eudragit S™; l'Eudragit L 100™; l'Eudragit L30D™; l'Eudragit L100-55 et l'Eudragit™ L et des mélanges de ceux-ci, ledit revêtement gastro-résistant comprenant en outre de préférence un agent glissant,
ou ledit revêtement gastro-résistant comprenant en outre de préférence un solvant organique, ledit solvant organique étant plus préférablement l'acétone.

22. Composition selon la revendication 4,
ledit sous-revêtement comprend en outre un solvant aqueux, ou
ledit sous-revêtement comprenant de l'Opadry II HP.

23. Composition selon l'une quelconque des revendications 1 à 4 ou 8 à 22, ledit substrat comprenant en outre au moins l'un parmi un liant, un tensioactif, une charge, un agent de solubilisation et un agent alcalinisant.

24. Composition selon la revendication 23, ledit liant comprenant un polymère hydrophile soluble dans l'eau,
ledit polymère étant sélectionné de préférence dans le groupe constitué par la Povidone (PVP : polyvinyl pyrrolidone), l'HPC de faible poids moléculaire (hydroxypropyl cellulose), l'HPMC de faible poids moléculaire (hydroxypropyl méthylcellulose), la carboxy méthyl cellulose de faible poids moléculaire, l'éthylcellulose, la gélatine, l'oxyde de polyéthylène, l'acacia, la dextrine, le silicate d'aluminium et de magnésium, l'amidon et les polyméthacrylates, et un mélange de ceux-ci, ledit liant étant plus préférablement l'hydroxypropyl méthylcellulose (HPMC).

25. Composition selon la revendication 23, ledit tensioactif comprenant du polysorbate 80 (Tween 80).

26. Composition selon la revendication 23, ladite charge étant sélectionnée dans le groupe constitué par un sucre ; le phosphate dicalcique ; un alcool de sucre ; l'amidon de maïs, l'amidon de pomme de terre, la carboxyméthylcellulose de sodium, l'éthylcellulose et l'acétate de cellulose, et un mélange de ceux-ci,
ledit sucre étant sélectionné de préférence dans le groupe constitué par le lactose, le glucose, le fructose et le saccharose, ladite charge étant plus préférablement le lactose, ou
ledit alcool de sucre étant sélectionné de préférence dans le groupe constitué par le sorbitol,
le mannitol, le maltitol, le lactitol, le xylitol, l'isomalt, l'érythritol et l'hydrolysat d'amidon hydrogéné.

27. Composition selon la revendication 23, ledit agent alcalinisant étant sélectionné dans le groupe constitué par le stéarate de sodium, la méglumine, le phosphate disodique et l'ammoniac,
ledit agent alcalinisant étant de préférence le stéarate de sodium, ou
ledit agent alcalinisant étant de préférence la méglumine.

28. Composition selon la revendication 1, au moins une partie de ladite multiplicité d'unités comprimées s'étant séparée lors de l'entrée dans le tractus gastro-intestinal.

29. Composition selon l'une quelconque des revendications 1 à 4 ou 8 à 28, ledit absorbeur de contrainte étant sélectionné dans le groupe constitué par les polysaccharides ou les polysaccharides réticulés, l'amidon, la cellulose microcristalline, l'éthylcellulose, les peptides ou les peptides réticulés, les protéines ou les protéines réticulées, la gélatine ou la gélatine réticulée, la gélatine hydrolysée ou la gélatine hydrolysée réticulée, le collagène ou le collagène réticulé, la cellulose modifiée, l'acide polyacrylique ou l'acide polyacrylique réticulé, les polyvinyles ou les polyvinyles réticulés, le polyacrylate et ses copolymères, et des mélanges de ceux-ci,
ledit polysaccharide réticulé étant sélectionné de préférence dans le groupe constitué par les sels métalliques insolubles ou les dérivés réticulés d'alginate, la pectine, la gomme xanthane, la gomme de guar, la gomme adragante et la gomme de caroube, le carraghénane, les sels métalliques de ceux-ci, et les dérivés réticulés de manière covalente de ceux-ci, ou
ladite cellulose modifiée étant sélectionnée de préférence dans le groupe constitué par les dérivés réticulés d'hydroxypropylcellulose, l'hydroxypropylméthylcellulose, l'hydroxyéthylcellulose, la méthylcellulose, la carboxyméthylcellulose et les sels métalliques de la carboxyméthylcellulose, et des mélanges de ceux-ci, ou
ledit absorbeur de contrainte de préférence étant la cellulose microcristalline.
